# EUROPÄISCHE PATENTSCHRIFT

(11) **EP 1 366 812 B1**
(45) Veröffentlichungstag und Bekanntmachung des Hinweises auf die Patenterteilung: **18.02.2009**
(21) Anmeldenummer: 02012040.8
(22) Anmeldetag: 31.05.2002
(51) Int. Cl.: B01J 23/46, B01J 35/10, C07C 209/72

(54) **Geträgerter Rutheniumkatalysator und Verfahren zur Hydrierung eines aromatischen Amins in Gegenwart dieses Katalysators**
Supported Ruthenium catalyst and process for hydrogenation of aromatic amines in presence of this catalyst
Catalyseur supporté à base de ruthenium et procédé d'hydrogénation d'amines aromatiques en présence de tel catalyseur

(43) Veröffentlichungstag der Anmeldung: 03.12.2003
(73) Patentinhaber: Evonik Degussa GmbH, 45128 Essen (DE)
(72) Erfinder: Stochniol, Guido, Dr., 63571 Gelnhausen (DE); Jäger, Bernd, Dr., 64297 Darmstadt (DE); Haas, Thomas, Dr., 60322 Frankfurt (DE); Finke, Norbert, Dr., 45739 Oer-Erkenschwick (DE); Burkhardt, Werner, 63636 Brachttal (DE); Grunert, Jürgen, 63579 Freigericht (DE)

(56) Entgegenhaltungen:
- EP-A- 0 324 190
- EP-A- 0 813 906
- EP-A- 0 814 098
- US-A- 5 110 779
- ERTL ET AL. (EDS.): "Handbook of Heterogeneous Catalysis, 2nd Edition, Vol. 2" WILEY-VCH , 3.1.3.10.2 * Seite 983 *
- PIERRE GALLEZOT: "Encyclopedie of Catalysis, Chapter Hydrogenation- Heterogeneous" WILEY , 2.3.4 Gefunden im Internet: URL:http://www.mrw.interscience.wiley.com/ emrw/9780471227618/enccat/article/eoc114/c ur rent/html#eoc114-sec1-0002> * Absatz [2.3.4] *

## Beschreibung

Die Erfindung richtet sich auf ein Verfahren zur Hydrierung eines aromatischen Amins, das mindestens eine an einem aromatischen Kern gebundene Aminogruppe aufweist, wobei die Hydrierung mit Wasserstoff in Gegenwart eines geträgerten Katalysators, welcher als Aktivmetall Ruthenium enthält, erfolgt. Der im erfindungsgemäßen Verfahren zu verwendende Katalysator enthält einen Katalysatorträger mit einer speziellen Eigenschaftskombination. In besonderer Weise richtet sich die Erfindung auf ein Verfahren zur Hydrierung von Methylendianilin (MDA) zu Bispara-aminocyclohexylmethan (PACM) mit einem Gehalt des trans-trans Isomeren hiervon im Bereich von insbesondere 15 bis 25 %.

Die bei der katalytischen Hydrierung von aromatischen Aminen erhältlichen cycloaliphatischen Amine, wie unsubstituierte oder substituierte Cyclohexylamine und Dicyclohexylamine, finden Verwendung bei der Herstellung von Polyamid- und Polyurethanharzen, als Härter für Epoxidharze sowie als Rohstoffe zur Herstellung von Kunststoff- und Kautschukadditiven sowie Korrosionsinhibitoren.

Es ist bekannt, cycloaliphatische Amine mit einer oder mehreren Aminogruppen durch katalytische Hydrierung der entsprechenden ein- oder mehrkernigen aromatischen Amine mit einer oder mehreren Aminogruppen und gegebenenfalls weiteren Substituenten herzustellen. Die Hydrierung derartiger Amine zu den entsprechenden cycloaliphatischen Aminen erfolgt, wie aus zahlreichen Dokumenten hervorgeht, vielfach unter Verwendung von geträgerten Katalysatoren.

So wird im Verfahren gemäß US-Patent 2,606,925 Bis-(4-aminophenyl-methan, nachfolgend vereinfacht als Methylendianilin oder MDA bezeichnet, in Gegenwart eines Ruthenium-Trägerkatalystators, wobei als Träger Aktivkohle, Aluminiumoxid und Kieselgur genannt werden zu Bis-(4-aminocyclohexyl-methan, nachfolgend vereinfacht als Bispara-aminocyclohexylmethan oder PACM bezeichnet, hydriert. PACM wird hierbei in Form des cis-cis-, cis-trans- und trans-trans-Isomeren gebildet. Hydriertemperaturen oberhalb 150°C oder verlängerte Reaktionszeiten bei der Hydrierung führen zu einem erhöhten Anteil des trans-trans-Isomeren. Diesem Dokument lassen sich keine Hinweise entnehmen, ob und in welcher Weise die Auswahl des Katalysators bzw. Katalysatorträgers die Isomerenverteilung beeinflusst.

Im Bemühen das thermodynamisch stabilere trans-trans-Isomere von PACM zu erhalten, wird die Hydrierung gemäß den US-Patenten 3,155,724 und 3,347,917 unter Verwendung eines Ruthenium-Trägerkatalysators in Gegenwart von Ammoniak durchgeführt. Die gattungsgemäße Hydrierung lässt sich gemäß US-Patent 3,914,307 auch dadurch verbessern, dass das zu hydrierende Reaktionsgemisch zusätzlich ein polyheterocyclisches Amin als Co-Katalysator enthält. Hinweise über das Isomerenverhältnis bei der Hydrierung mehrkerniger aromatischer Amine wie MDA zu PACM sowie über einen Einfluss der Eigenschaften des Trägermaterials lassen sich den letztgenannten Dokumenten nicht entnehmen.

Das US-Patent 5,360,934 lehrt das gattungsgemäße Verfahren, wobei jedoch ein Rhodium enthaltender Trägerkatalysator verwendet wird. Als Aktivmetall kann auch Ruthenium anwesend sein. Nach der Lehre dieses Dokuments hängt die Katalysatoraktivität in erheblichem Umfang von der Modifikation und des als Träger eingesetzten Aluminiumoxids ab. Hiernach sind Katalysatoren mit Delta-, Theta- und Kappa-Aluminiumoxid als Trägermaterial aktiver als ein Katalysator mit handelsüblichem Gamma-Aluminiumoxid als Trägermaterial.

Gemäß EP 0 066 211 A1 lässt sich Dianilinomethan in PACM mit einem trans-trans-Isomerenanteil des PACM im Bereich von 15 bis 40 Gew.-%, insbesondere 18,5 bis 23,5 Gew.-% überführen, indem die Hydrierung in Gegenwart eines trägerfreien Rutheniumkatalysators durchgeführt wird. Nachteilig an diesem Verfahren sind der hohe erforderliche Hydrierdruck und die hohe Reaktionstemperatur, ferner der größere Aufwand zur Abrennung des Rutheniumkatalysators aus dem Reaktionsgemisch.

Gemäß EP-Patent 0 324 190 lässt sich PACM mit dem zuvor genannten niedrigen trans-trans-Isomerenanteil durch Hydrierung von MDA in Gegenwart eines trägergebunden Rutheniumkatalysators gewinnen. Die Hydrierung erfolgt bei 100 bis 190 °C und einem Druck von 5 bis 35 MPa, wobei in den beispielhaften Ausführungsformen zwar die Temperatur im unteren Temperaturbereich lag, der Wasserstoffdruck jedoch mit 30 MPa im oberen des zuvor genannten Druckbereiches. Das Trägermaterial des in diesem Verfahren eingesetzten Katalysators ist gekennzeichnet durch einen BET-Oberflächenbereich von 70 bis 280 m²/g und einen mittleren Porendurchmesser dₚ von 1 bis 32 nm; die Eindringtiefe des Rutheniums beträgt mindestens 50 µm, insbesondere 100 bis 300 µm; der Rutheniumgehalt wird mit 0,1 bis 5 Gew.-%, insbesondere 0,5 bis 3 Gew.-% angegeben. Ein Nachteil dieses Verfahrens ist der nach wie vor in der Praxis erforderliche hohe Wasserstoff-Hydrierdruck.

Im Verfahren gemäß EP 0 813 906 A2 lassen sich organische Verbindungen, darunter auch aromatische Verbindungen, in welchen mindestens eine Aminogruppe an einem aromatischen Kern gebunden ist, unter Verwendung eines Ruthenium-Trägerkatalysators hydrieren. Der Katalysator kann als Aktivmetall zusätzlich zu Ruthenium andere Metalle aus der ersten, siebten oder achten Nebengruppe des Periodensystems enthalten. Gegenüber dem Trägermaterial des zuvor gewürdigten EP-Patents 0 324 190 weist das Trägermaterial hier eine BET-Oberfläche von höchstens 30 m²/g und einen mittleren Porendurchmesser von mindestens 50 nm auf. Gekennzeichnet ist der hier verwendete Katalysator zudem durch ein Verhältnis der Oberfläche des Aktivmetalls und Oberfläche des Katalysatorträgers von kleiner 0,05. Bei den makroporösen Trägermaterialien mit einem mittleren Porendurchmesser von vorzugsweise 500 nm bis ungefähr 50 µm handelt es sich bevorzugt um Aluminiumoxid und Zirkoniumdioxid. Einzelheiten zur Hydrierung von MDA zu PACM sind diesem Dokument nicht zu entnehmen. Insbesondere führt die Hydrierung aromatischer Verbindungen, wie 4-Alkalyl-substituierten Phenolen, überwiegend zu transcycloaliphatischen Verbindungen. Im Gegensatz hierzu stellten sich die Erfinder der vorliegenden Anmeldung die Aufgabe, substituierte aromatische Amine in cycloaliphatische Amine mit niedrigem trans-Anteil zu überführen.

Ein ähnliches Verfahren wie jenes der EP 0 813 906 A2 lehrt die EP 0 814 098 A2: Als Trägermaterial für den trägergebundenen Ruthenium-Hydrierkatalysator für die Hydrierung aromatischer Amine zu cycloaliphatischen Aminen werden hier solche Materialien verwendet, deren Porenvolumen zu 10 bis 50% aus Makroporen mit einem Porendurchmesser im Bereich von 50 nm bis 10.000 nm und zu 50 bis 90 % aus Mesoporen mit einem Porendurchmesser im Bereich von 2 bis 50 nm gebildet wird. Die BET-Oberfläche des Trägers wird mit 50 bis 500 m²/g, insbesondere 200 bis 350 m²/g angegeben. Das Verhältnis der Oberfläche des Aktivmetalls und des Trägers soll weniger als 0,3, insbesondere weniger als 0,1 betragen. Angaben über die Aktivität derartiger Katalysatoren sowie das Isomerenverhältnis bei der Hydrierung von MDA zu PACM lassen sich diesem Dokument nicht entnehmen. Allerdings wird auch hier unter Hinweis auf die Hydrierung 4-substituierte Phenole auf die überwiegende Bildung der trans-Isomeren hingewiesen.

Schließlich wird in der EP 0 873 300 B1 vorgeschlagen, die Hydrierung aromatischer Amine, wie Bis-p-aminophenylmethan, mit einem trägergebundenen Ruthenium als Aktivmetall enthaltenden Katalysator durchzuführen, wobei der Träger einen mittleren Porendurchmesser von mindestens 0,1 µm, insbesondere mindestens 0,5 µm und eine Oberfläche von höchstens 15 m²/g, vorzugsweise 0,05 bis 5 m²/g aufweist. Nach diesem Verfahren lassen sich cycloaliphatische Amine mit hoher Selektivität und ohne die Bildung von Desaminierungsprodukten oder teilhydrierten Dimerisierungsprodukten gewinnen. Hinweise zum trans-trans-Isomerenanteil lassen sich diesem Dokument nicht entnehmen.

Wie aus einer Publikation von G.F. Allen (Chem. Ind. (Dekker) (1988), 33 Catal. Org. Reakt., 323 - 338) bekannt ist, steigt mit zunehmendem Umsatz an Methylendianilin der trans-trans-Isomerenanteil von PACM an. Die beiden zuvor genannten Verfahren richten sich jedoch auf den Erhalt eines hohen Umsatzes.

Schließlich lehrt die EP 0 639 403 A2, dass sich Bis -paraaminocyclohexylmethan durch Hydrierung von Methylendianilin in vorteilhafter Weise mit einem Ruthenium oder Rhodium enthaltenden Trägerkatalysator erzeugen lässt, wobei die Schichtdicke der Aktivmetalle auf dem Träger 5 bis 150 µm, vorzugsweise 10 bis 80 µm beträgt. Bei dem Trägermaterial handelt es sich um eine kalzinierte und oberflächlich rehydratisierte Übergangstonerde mit einem spezifizierten pH-Wert. Der Träger weist eine BET-Oberfläche von mindestens 70 m²/g auf und eine offene Porosität von mindestens 0,1 ml/g auf. Hinweise über die Porenverteilung lassen sich diesem Dokument nicht entnehmen. Ein Vorteil dieses Verfahrens besteht darin, dass auch nach längerer Betriebszeit der Anteil an trans-trans-Isomer von PACM im Bereich von etwa 20 bis 25 % liegt.

Nachteil dieses Verfahrens sind jedoch der erhöhte Aufwand, einen Gleichgewichts- pH-Wert einzustellen, der hohe apparative Aufwand um den in den Beispielen genannten hohen Wasserstoffdruck (300 bar) anwenden zu können, und die beschränkte Trägerauswahl.

Aufgabe der vorliegenden Erfindung ist es, ein weiteres Verfahren zur Hydrierung aromatischer Amine in Gegenwart eines rutheniumenthaltenen Trägerkatalysators aufzuzeigen, mit dem die gewünschten cycloaliphatischen Amine in hoher Selektivität gewonnen werden können. Eine weitere Aufgabe der Erfindung richtet sich auf die Bereitstellung eines weiteren Verfahrens zur Herstellung von Bis-paraaminocyclohexylmethan durch katalytische Hydrierung von Methylendianilin, wobei der trans-trans-Isomerenanteil des PACM weniger als 30 %, insbesondere 15 bis 25 % betragen sollte. Eine weitere Aufgabe richtet sich darauf, dass trotz eines hohen Umsatzes ein niedriger trans-trans-Anteil erhalten bleibt. Gemäß einer weiteren Aufgabe sollte der im Verfahren eingesetzte Katalysator eine hohe Standzeit aufweisen und die Isomerenverteilung auch nach längerer Betriebsdauer im wesentlichen unverändert belassen.

Diese und weitere Aufgaben wie sie sich aus der weiteren Beschreibung ergeben, lassen sich durch das erfindungsgemäße Verfahren lösen.

Gefunden wurde demgemäß ein Verfahren zur Hydrierung eines aromatischen Amins, das mindestens eine an einen aromatischen Kern gebundene Aminogruppe aufweist, umfassend Umsetzung des aromatischen Amins mit Wasserstoff in Gegenwart eines geträgerten Katalysators, der als Aktivmetall Ruthenium alleine oder zusammen mit mindestens einem Metall des I., VII. oder VIII. Nebengruppe des Periodensystems in einer Menge von 0,01 bis 20 Gew.%, insbesondere 0,2 bis 3 Gew.-% Aktivmetalle, bezogen auf den geträgerten Katalysator, auf einen Träger aufgebracht enthält, dadurch gekennzeichnet, dass der Katalysatorträger eine BET-Oberfläche im Bereich von größer 30 m²/g bis kleiner 70m²/g aufweist und mehr als 50% des Porenvolumens des Katalysatorträgers Makroporen mit einem Porendurchmesser von größer 50 nm und weniger als 50% Mesoporen mit einem Porendurchmesser von 2 bis 50 nm sind und dass das auf den Katalysator aufgebrachte Aktivmetall eine Eindringtiefe in den Träger im Bereich von 20 bis 500 µm, insbesondere 25 bis 250 µm, aufweist.

Die Unteransprüche richten sich auf bevorzugte Ausführungsformen des erfindungsgemäßen Verfahrens.

Im Hinblick auf den zuvor eingehend gewürdigten Stand der Technik, insbesondere der EP 0 814 098 A2, war es überraschend, dass ein Katalysatorträger mit einer spezifischen Oberfläche im Bereich von größer 30 m²/g bis kleiner 70 m²/g im gattungsgemäßen Verfahren besonders wirksam ist, wenn mehr als 50 % des Porenvolumens aus Makroporen und weniger als 50 % des Porenvolumens aus Mesoporen gebildet wird. Wesentlich ist somit nicht die BET-Oberfläche allein oder die Porenverteilung allein, sondern die Kombination dieser beider Merkmale. Überraschend ist die erfinderische Eigenschaftskombination des Trägermaterials auch deshalb, weil in der zuvor gewürdigten EP 0 324 190 B bei einer BET-Oberfläche von mindestens 70 m²/g das Porenvolumen im wesentlichen ausschließlich von Mesoporen mit einem mittleren Porendurchmesser von 1 bis 32 nm gebildet werden sollte. Schließlich unterscheidet sich der im Verfahren der vorliegenden Erfindung gemäß zu verwendende Katalysator in prinzipieller Weise von dem in der EP 0 813 906 A2 genannten Katalysator, weil der Katalysatorträger im vorbekannten Verfahren zwar makroporös ist, die BET- Oberfläche jedoch höchstens 30 m²/g und vorzugsweise höchstens 15 m²/g betragen soll. Das Verhältnis der Oberfläche des Aktivmetalls und des Katalysatorträgers liegt im Bereich von 0,01 bis 0,5, insbesondere 0,03 bis 0,3. Überraschender Weise führt auch ein geringes Oberflächenverhältnis des Aktivmetalls, bestimmt durch CO-Chemisorption, und des Katalysatorträgers, bestimmt nach BET, von 0,03 bis 0,06 bei erfindungsgemäß zu verwendenden Katalysator unter milden Hydrierbedingungen zu einer hohen Katalysatoraktivität.

### Aromatische Amine:

Mit dem erfindungsgemäßen Verfahren können aromatische Amine, in denen mindestens eine Aminogruppe an einen aromatischen Kern gebunden ist, zu den entsprechenden cycloaliphatischen Verbindungen hydriert werden. Dabei können die aromatischen Verbindungen einkernige oder mehrkernige aromatische Verbindungen sein. Vorzugsweise sind die aromatischen Verbindungen aromatische Amine oder Diamine oder Triamine. Die aromatischen Amine können an dem oder den aromatischen Kernen oder/und an der Aminogruppe substituiert sein, beispielsweise durch einen oder mehrere Alkyl- und/oder Alkoxyreste, vorzugsweise C₁₋₂₀-Alkyl- und/oder C₁₋₂₀-Alkoxyreste. Besonders bevorzugte Substituenten sind C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, insbesondere Methoxy, Ethoxy, Propoxy und Butoxy bevorzugt. Der oder die aromatischen Kerne sowie die Alkyl- und Alkoxyreste können gegebenenfalls durch Halogenatome, insbesondere Fluoratome, substituiert sein oder andere geeignete inerte oder hydrierbare Substituenten aufweisen.

Das aromatische Amin in dem mindestens eine Aminogruppe an einen aromatischen Kern gebunden ist, kann auch mehrere aromatische Kerne aufweisen, die über einen zweiwertigen Kohlenwasserstoffrest, wie eine Methylengruppe oder Ethylengruppe verknüpft sind. Der verknüpfende Rest kann einen oder mehrere Alkylsubstituenten, insbesondere C₁₋₂₀-Alkylreste, bevorzugt einen oder mehrere Methyl-, Ethyl-, n- oder iso-Propyl-, n- oder sek.-Butyl- oder tert.-Butylreste aufweisen.

Besonders bevorzugte aromatische Amine sind Anilin, Naphthylamin, Bis(p-aminophenyl)methan, sowie Isomere davon, also 2,4'- und 2,2'-Diamino-diphenylmethan, Bis(p-aminophenyl)amine, 2,2-Bis(p-aminophenyl)propan, wobei einer oder beide aromatischen Kerne eine weitere Aminogruppen und/oder eine C₁-bis C₃-Alkyl- oder Alkoxygruppe aufweisen können.

### Katalysatoren:

Die erfindungsgemäß zu verwendenden Trägerkatalysatoren können technisch hergestellt werden durch Auftragen von Ruthenium und gegebenenfalls mindestens einem Metall der I-, VII- oder VIII-Nebengruppe auf einen geeigneten Träger. Die Auftragung kann durch Aufsprühen von wässrigen Metallsalzlösungen, wie Rutheniumsalzlösungen, auf den Träger oder durch andere geeignete Verfahren erreicht werden. Als Salze zur Herstellung der Rutheniumsalzlösungen sowie Lösungen von Metallsalzen von Elementen der I-, VII- oder VIII- Nebengruppe eignen sich die Nitrate, Nitrosylnitrate, Halogenide, Carbonate, Carboxylate, Acetylacetonate, Chlorkomplexe, Nitrokomplexe oder Aminkomplexe der entsprechende Metalle; bevorzugt sind Nitrate und Nitrosylnitrate.

Bei Katalysatoren, die neben Ruthenium noch weitere Metalle auf den Träger aufgetragen enthalten, können die Metallsalze bzw. Metallsalzlösungen gleichzeitig oder nacheinander aufgebracht werden.

Die mit einem Rutheniumsalz bzw. zusätzlich weiteren Metallsalzlösungen beschichteten Träger werden getrocknet, vorzugsweise bei Temperaturen zwischen 100°C und 150°C, und wahlweise bei Temperaturen zwischen 200°C und 600°C kalziniert. Anschließend werden die beschichteten Träger durch Behandlung der beschichteten Träger in einem Gasstrom, der freien Wasserstoff enthält, bei Temperaturen zwischen 30 und 600°C, vorzugsweise zwischen 150 und 400 °C aktiviert. Der Gasstrom besteht vorzugsweise aus 50 bis 100 Vol.-% H₂ und 0 bis 50 Vol.-% N₂.

Werden auf den Trägern neben Ruthenium noch ein oder mehrere andere Metalle der I-, VII oder VIII-Nebengruppe aufgetragen, und erfolgt das Auftragen nacheinander, so kann der Träger nach jedem Auftragen bei Temperaturen zwischen 100 und 150°C getrocknet werden und wahlweise bei Temperaturen zwischen 200 und 600 °C kalziniert werden. Dabei kann die Reihenfolge, in der die Metallsalzlösungen aufgetragen werden, beliebig gewählt werden.

Gemäß einer bevorzugten Ausführung erfolgt die Beschichtung des Trägers durch Aufsprühen einer Metallsalzlösung bei erhöhter Temperatur, insbesondere oberhalb 50°C und besonders bevorzugt bei 80 bis 150°C, so dass bereits während der Beschichtung das Lösungsmittel zumindest teilweise verdampft wird und die Eindringtiefe der katalytisch wirksamen Metalle limitiert wird. Vorzugsweise liegt die Eindringtiefe im Bereich von 25 bis 250 µm, insbesondere 25 bis 150 µm und besonders bevorzugt bei 50 bis 120 µm.

Die Rutheniumsalzlösung und ggf. weitere Metallsalzlösungen werden in einer solchen Menge auf den oder die Träger aufgebracht, dass 0,01 bis 20 Gew.-%, bezogen auf das Gesamtgewicht des Katalysators, an Ruthenium und gegebenenfalls anderen Metallen der I., VII. oder VIII. Nebengruppe auf den Träger aufgebracht werden. Vorzugsweise beträgt die Menge an Aktivmetallen 0,2 bis 15 Gew.-%, insbesondere etwa 0,2 bis 3 Gew.-%, wobei der Rutheniumgehalt den Gehalt der anderen Metalle zweckmäßiger Weise übersteigt.

### Trägermaterialien:

Die Trägermaterialien der erfindungsgemäß zu verwendenden Katalysatoren weisen eine spezifische BET-Oberfläche (bestimmt nach DIN 66131 mit N₂) im Bereich von größer 30 m²/g und kleiner 70 m²/g auf.

Der Träger enthält Makroporen mit einem Porendurchmesser von größer 50nm. Der Durchmesser der Makroporen liegt insbesondere im Bereich von 50 bis 50.000 nm, vielfach aber im Bereich von 50 bis 10.000 nm. Soweit der Träger auch Mesoporen umfasst, werden hierunter Poren im Bereich von 2 bis 50 nm verstanden. Mindestens 50 % des Porenvolumens wird aus Makroporen und weniger als 50 % aus Mesoporen gebildet. Bevorzugte Träger enthalten Makroporen in einer Menge von 55 bis 85 % des Porenvolumens,und 15 bis 45 % des Porenvolumens nehmen Mesoporen ein. In besonders bevorzugten Trägern nehmen Mesoporen etwa 25 bis 45 % des Porenvolumens ein und Makroporen den Rest des Porenvolumens. Mikroporen mit einem Porendurchmesser von kleiner 2 nm sind, sofern anwesend, nur in einer Menge von weniger als 10 % des Porenvolumens, insbesondere weniger als 1 % anwesend.

Die Modifikation des Trägers kann einheitlich oder gemischt sein, so dass die Porenverteilung monomodal, bimodal oder trimodal sein kann.

Prinzipiell sind alle bekannten Trägermaterialien für Hydrierkatalysatoren verwendbar, soweit sie die anspruchsgemäße BET-Oberfläche, Porengröße und Porenverteilung aufweisen. Geeignet sind oxidische, silikatische und nitridische Träger und zwar in ein- oder mehrphasiger kristalliner oder röntgenamorpher oder gemischter Struktur.

Die Träger können ferner in bekannter Weise mit Alkali- oder/und Erdalkaliverbindungen und/oder mit Metallen der Lanthanidreihe modifiziert sein.

Beispielhafte Träger sind Oxide aus der Reihe Al₂O₂, TiO₂, ZrO₂, SiO₂, MgO und ZnO, ferner Mischoxide, wie Spinelle, z.B. MgAl₂O₄. Auch Alumosilikate und Aktivkohle sind geeignet, sofern diese Träger die anspruchsgemäße Eigenschaftskombination aufweisen. Besonders bevorzugt sind die Oxide Al₂O₃ und TiO₂.

### Hydrierbedingungen:

Die Hydrierung wird bei einer Temperatur im Bereich von 20 bis 250°C, insbesondere bei unter 200°C und einem wirksamen H₂-Partialdruck, im Bereich von etwa 1 bis 30 MPa , bevorzugt unter 10 MPa in einem kontinuierlich oder diskontinuierlich zu betreibenden Suspensions- oder Festbett-Hydrierreaktor durchgeführt. Die Aktivität der erfindungsgemäßen Katalysatoren ermöglicht es, die Hydrierung unter milden Bedingungen, insbesondere bei einer Temperatur im Bereich von 50 bis 150°C, insbesondere 70 bis 120°C und einem H₂-Druck im Bereich von 3 bis 10 MPa durchzuführen, so dass technisch weniger aufwendige Reaktoren verwendet werden können, wodurch die Wirtschaftlichkeit des Verfahrens steigt.

Die Hydrierung kann in An- oder Abwesenheit eines geeigneten Lösungsmittels durchgeführt werden. Bevorzugt ist ein Lösungsmittel anwesend, und zwar in einer Menge von etwa 10 bis 90 Gew.-%, bezogen auf die Lösung des zu hydrierenden aromatischen Amins.

Geeignete Lösungmittel sind beispielhaft: primäre, sekundäre und tertiäre ein- oder mehrwertige Alkohole, wie Methanol, Ethanol, n- und i-Propanol, n-, sek.-, und tert.-Butanol, Ethylenglykol, Ethylenglykolmono (C₁-C₄) alkylether; lineare Ether, wie Ethylenglykoldi (C₁-C₃) alkylether; cyclische Ether, wie Tetrahydrofuran und Dioxan,; Alkane, wie n- und iso-Alkane mit 4 bis 12 C-Atomen, z.B. n-Pentan, n-Hexan und Isooctan, und zyklische Alkane, wie Cyclohexan und Dekalin.

Die Hydrierung kann auch in Gegenwart von Ammoniak oder eines primären, sekundären oder tertiären Amins oder eines polyzyklischen Amins mit einem verbrückten N-Atom konnte durchgeführt werden. Zweckmäßiger Weise wird durch orientierende Versuche sichergestellt, dass unter den gewählten keine unerwünscht Isomerisierung im Falle von PACM also in Richtung eines höheren trans-trans-Anteils statt findet.

Lösungsmittel kann auch das Hydrierprodukt selbst sein, also ein cycloaliphatisches Amin sein.

Zur kontinuierlichen Hydrierung wird ein Festbettreaktor bevorzugt. Der Festbettreaktor kann als Blasenreaktor betrieben werden, bevorzugt wird aber eine Rieselbettfahrweise. Vorzugsweise wird ein Rieselbettreaktor mit einem LHSV-Wert im Bereich von 0,1 bis 5 h⁻¹ (=1 des zu hydrierenden aromatischen Amins pro l Festbettkatalysator und Stunde). Gemäß einer besonders bevorzugten Ausführungsform des erfindungsgemäßen Verfahrens wird ein Rohrbündelreaktor verwendet und dieser in Rieselbettfahrweise betrieben.

Ein weiterer Gegenstand der vorliegenden Erfindung ist der für das erfindungsgemäße Verfahren geeignete trägergebundene Katalysator. Gegenstand ist somit ein geträgerter Katalysator, der als Aktivmetall Ruthenium alleine oder zusammen mit mindestens einem Metall der I., VII. oder VIII. Nebengruppe des Periodensystems in einer Menge von 0,01 bis 20 Gew.% Aktivmetalle, bezogen auf den geträgerten Katalysator, auf einen Träger aufgebracht enthält, zur Hydrierung eines aromatischen Amins, das mindestens eine an einen aromatischen Kern gebundene Aminogruppe aufweist, dadurch gekennzeichnet, dass der Katalysatorträger eine BET-Oberfläche im Bereich von größer 30 m²/g bis kleiner 70m²/g aufweist und mehr als 50% des Porenvolumens des Katalysatorträgers Makroporen mit einem Porendurchmesser von größer 50 nm und weniger als 50% Mesoporen mit einem Porendurchmesser von 2 bis 50 nm sind. Das auf den Katalysator aufgebrachte Aktivmetall weist eine Eindringtiefe in den Träger im Bereich von 20 bis 500 µm, insbesondere 25 bis 250 µm, auf. Dieser Katalysator lässt sich auch für andere Hydrierungen, beispielsweise die Hydrierung von Phenolen und Nitrilen verwenden. Vorzugsweise übersteigt die Rutheniummenge die Menge der übrigen Aktivmetalle. Bevorzugt enthält der Katalysator 0,2 bis 3 Gew.-% Aktivmetalle, wobei insbesondere mindestens 90 % Ruthenium ist. Weitere Merkmale und bevorzugte Ausführungsform sind in Verbindung mit dem erfindungsgemäßen Verfahren offenbart worden.

### Beispiele:

### Herstellung des Katalysators:

### Beispiel 1:

Ein Aluminiumoxid-Formkörper(Extrudat, d= 3mm) mit einer BET-Oberfläche von ca.33 m²/g und einer bimodalen Porenverteilung mit einem Porenvolumen von 0,41 ml/g, wobei im wesentlichen keine Poren mit einem Durchmesser von 2 bis 50 nm festgestellt wurden, jedoch 100 % des Porenvolumens aus Makroporen mit einem Durchmesser im Bereich von 50 bis 10.000 nm bestand, wurde mit einer wässrigen Ruthenium-(III)-Nitrat-Lösung bei ca. 90 bis 100°C beschichtet, indem die Katalysatorlösung auf das in Bewegung befindliche Trägermaterial aufgesprüht wurde, wobei gleichzeitig Wasser verdampfte. Die Katalysatorlösung wies eine Konzentration von 5 % Metall, bezogen auf das Gewicht der Lösung, auf.

Der so beschichtete Träger wurde bei einer Temperatur von 120 bis 180 °C erhitzt und anschließend mit einem Gemisch aus 50 % H₂ und 50 % N₂ bei 200°C für 4 h reduziert. Der so hergestellt Katalysator enthielt 3 Gew.-% Ruthemium, bezogen auf das Gesamtgewicht des Katalysators. Die Eindringtiefe des Rutheniums betrug 70 - 90 µm. Das Verhältnis der Rutheniumoberfläche bestimmt durch CO-Chemisorption zur Oberfläche des unbeschichteten Trägermaterials bestimmt nach BET betrug etwa 0,05. Der Aluminiumoxid-Formkörper bestand im wesentlichen aus alpha- und gamma-Al₂O₃ (ca. 18 Gew.-%. SiO₂ und ca. 2 Gew.-% Alkali- und Erdalkalioxide Fe₂O₃ und Ti₂O.

### Beispiel 2:

Ein Aluminiumoxid-Formkörper (Extrudat, d= 3mm) ähnlicher Zusammensetzung wie der Träger des Beispiels 1 mit einer BET-Oberfläche von ca. 32 m²/g, trimodaler Porenverteilung und einem Porenvolumen von 0,58 ml/g wurde in analoger Weise wie in Beispiel 1 imprägniert. Das Porenvolumen des Trägermaterials resultierte aus 31 % Poren mit 2 bis 50 nm, 44 % Poren mit 50 bis 10.000 nm und 25 % Poren mit einem Porendurchmesser von größer 10.000 nm bis 5 µm. Der so hergestellte Katalysator enthielt wie Beispiel 1 3 Gew.-% Ruthenium, und die Eindringtiefe betrug 70 bis 90 µm.

### Beispiel 3:

Ein Aluminiumoxid-Formkörper (Extrudat d= 3mm) mit einer Oberfläche von ca. 54 m²/g wies bei trimodaler Porenverteilung ein Porenvolumen von 0,77 ml/g auf. 40 % des Porenvolumens resultierte aus Poren mit einem Durchmesser von 2 bis 50 nm, 60 % aus Poren mit einem Porendurchmesser von 50 bis 10.000 nm. Die Imprägnierung des Trägers, Kalzinierung und Reduktion des Katalysators erfolgten in gleicher weise wie bei Beispiel 1. Der so hergestellte Katalysator enthielt 3 Gew.-% Ruthenium bezogen auf das Gesamtgewicht des Katalysators. Die Eindringtiefe betrug 70 bis 90 nm. Der eingesetzte Aluminiumoxid-Formkörper umfasste die alpha-, theta- und gamma-Al₂O₃-Modifikationen.

### Beispiel 4:

Ein Aluminiumoxid-Formkörper (Kugeln mit 2 - 4 mm) mit einer BET-Oberfläche von ca. 35 m²/g wies bei monomodaler Porenverteilung ein Porenvolumen von 0,5 ml/g auf. 42 % des Porenvolumens wurde aus Mesoporen (2 bis 50 nm)und 58 % aus Makroporen (50 bis 10.000 nm) gebildet. Das Trägermaterial umfasste die theta- und gamma-Al₂O₃-Modifikationen. Die Imprägnierung, Kalzinierung und Reduktion erfolgten in gleicher Weise wie in Beispiel 1. Der so erhaltene trägergebundene Rutheniumkatalysator enthielt 3 Gew.-% Ruthenium, bezogen auf das Gesamtgewicht des Katalysators. Die Eindringtiefe des Rutheniums betrug 80 bis 120 µm.

### Vergleichsbeispiel 1:

Ein Titandioxid-Formkörper (Extrudat, d= 2mm) im wesentlichen bestehend aus einem Gemisch aus Rutil und Anatas mit einer BET-Oberfläche von 45 m²/g wies bei monomodaler Porenverteilung ein Porenvolumen von 0,35 ml/g auf. Das Porenvolumen wurde zu 100 % aus Mesoporen (2 bis 50 nm) gebildet. Der Formkörper wurde in analoger Weise wie in Beispiel 1 imprägniert, die Trocknung erfolgte jedoch bei 150 bis 160 °C und die anschließende Reduktion erfolgte bei 180 °C innerhalb von 4 h. Der so hergestellt Katalysator enthielt 3 Gew.-% Ruthenium bezogen auf das Gesamtgewicht des Katalysators. Die Eindringtiefe betrug 90 bis 120 µm.

### Vergleichsbeispiel 2:

Ein Aluminiumoxid-Formkörper (Extrudat D= 1,2 nm) aus im wesentlichen gamma-Al₂O₃ mit einer BET-Oberfläche von 220 m²/g hatte ein Porenvolumen von 0,65 ml/g, wobei 95 % des Porenvolumens aus Mesoporen (2 bis 50 nm) und 5 % des Porenvolumens aus Makroporen (50 bis 10.000 nm) gebildet wurden. Der Träger wurde mit einer wässrigen Ruthenium-(III)-nitrat-Lösung bei Raumtemperatur imprägniert. Die Katalysatorlösung besaß eine Konzentration von 5 % Metall, bezogen auf das Gewicht der Lösung. Der imprägnierte Träger wurde bei einer Temperatur von 150 bis 160 °C erhitzt und anschließend mit einem Gemisch aus 50 % H₂ und 50 % N₂ bei 180 °C für 4 h reduziert. Der so hergestellte Katalysator enthielt 5 Gew.-% Ruthenium bezogen auf das Gesamtgewicht des Katalysators. Die Eindringtiefe betrug bis 600 µm.

### Durchführung der Hydrierreaktion

### Beispiele 5 bis 8 und Vergleichsbeispiele 3 und 4.

Jeweils 30 ml eines Ruthenium-Trägerkatalysators gemäß einem der Beispiele 1 bis 4 oder Vergleichsbeispiele 1 bis 2 wurde in einen mit in einem Außenmantel beheizten Rohrreaktor eingefüllt: der Reaktor war mit einer Abscheidevorrichtung ausgestattet. Der Festbettreaktor wurde zunächst bei 90 °C mit Wasserstoff beaufschlagt und dann mit einer Lösung aus 20 Vol.-% 4,4'-Methylendianilin und 80 Vol.-% Tetrahydrofuran beschickt. Bei einem Wasserstoffpartialdruck von 8 Mpa wurde hydriert. Die Katalysatorbelastung betrug bei dieser Rieselbettfahrweise 0,43 [h⁻¹]. Während des Berieselns wurde die Temperatur bis auf 110 °C erhöht.

Die nachfolgende Tabelle zeigt Betriebsdaten und Ergebnisse darunter die Reaktionstemperatur, den verwendeten erfindungsgemäßen oder nichterfindungsgemäßen Katalysator, den Umsatz an 4,4'-Methylendianilin (MDA), den MDA-Umsatz pro g Ru/h sowie den trans-trans-Isomerenanteil. Die Prozentangaben beziehen sich auf gaschromatographisch ermittelte Flächenprozente.

Aus den Ergebnissen der erfindungsgemäßen Beispiele folgt die außergewöhnliche Aktivität der erfindungsgemäß zu verwendenden Ru-Trägerkatalysatoren. Unter Verwendung der erfindungsgemäßen Katalysatoren erhält man PACM mit einem trans-trans-Isomerenanteil im Bereich von etwa 15 - 25 %.

**Tabelle:**

| Bsp.-Nr. | Katalysator | Hydriertemperatur (°C) | MDA-Umsatz (%) | % MDA-Umsatz / gRu·h | gPACM/gRu·h | PACM trans-trans-Isomere (%) |
|---|---|---|---|---|---|---|
| B 5/1 | B 1 | 102 | 97,4 | 170 | 3,3 | 18 |
| /2 | B 1 | 105 | 99,4 | 174 | 3,5 | 20 |
| /3 | B 1 | 110 | 99,8 | 175 | 3,6 | 25 |
| B 6/1 | B 2 | 94 | 99,8 | 169 | 3,0 | 15 |
| /2 | B 2 | 102 | 100 | 170 | 3,7 | 21 |
| B 7 | B 3 | 100 | 99 | 184 | 4,5 | 16 |
| B 8/1 | B 4 | 98 | 94,2 | 149 | 3,9 | 22 |
| VB 3/1 | VB 1 | 100 | 96,3 | 113 | 2,0 | 22 |
| /2 | VB 1 | 105 | 98,4 | 115 | 2,3 | 25 |
| VB 4 | VB 2 | 94 | 93 | 89 | 1,8 | 20 |

## Patentansprüche

1. Verfahren zur Hydrierung eines aromatischen Amins, das mindestens eine an einen aromatischen Kern gebundene Aminogruppe aufweist, umfassend Umsetzung des aromatischen Amins mit Wasserstoff in Gegenwart eines geträgerten Katalysators, der als Aktivmetall Ruthenium alleine oder zusammen mit mindestens einem Metall der I., VII. oder VIII. Nebengruppe des Periodensystems in einer Menge von 0,01 bis 20 Gew.% Aktivmetalle, bezogen auf den geträgerten Katalysator, auf einen Träger aufgebracht enthält,
**dadurch gekennzeichnet,**
**dass** der Katalysatorträger eine BET-Oberfläche im Bereich von größer 30 m²/g bis kleiner 70m²/g aufweist und mehr als 50% des Porenvolumens des Katalysatorträgers Makroporen mit einem Porendurchmesser von größer 50 nm und weniger als 50% Mesoporen mit einem Porendurchmesser von 2 bis 50 nm sind und dass das auf den Katalysator aufgebrachte Aktivmetall eine Eindringtiefe in den Träger im Bereich von 20 bis 500 µm, insbesondere 25 bis 250 µm, aufweist.

2. Verfahren nach Anspruch 1,
**dadurch gekennzeichnet,**
**dass** das Verhältnis der Oberflächen des Aktivmetalls, bestimmt durch CO-Pulschemisorption, und des Katalysatorträgers, bestimmt nach BET, größer als 0,01, insbesondere 0,03 bis 0,3, ist.

3. Verfahren nach Anspruch 1 oder 2,
**dadurch gekennzeichnet,**
**dass** das Trägermaterial ausgewählt ist aus der Reihe der kristallinen und amorphen Oxide und Silikate, insbesondere ausgewählt aus der Reihe Al₂O₃, SiO₂, TiO₂, ZrO₂, MgO, ZnO und Alumosilikate.

4. Verfahren nach einem der Ansprüche 1 bis 3,
**dadurch gekennzeichnet,**
**dass** der Katalysatorträger eine BET-Oberfläche im Bereich von 32 bis 67 m²/g aufweist, die Eindringtiefe der Aktivmetalle im Bereich von 50 bis 200 µm und die Ru-Menge im Bereich von 0,2 bis 3 Gew.%, bezogen auf den Katalysator, liegt und mindestens 55% des Porenvolumens des Katalysatorträgers aus Makroporen und weniger als 45% aus Mesoporen gebildet wird.

5. Verfahren nach einem der Ansprüche 1 bis 4,
**dadurch gekennzeichnet,**
**dass** man ein 4,4'-Diamino-diphenyl-(C₁ bis C₄) alkan und/oder ein 2,4'- oder 2,2'-Isomeres hiervon, insbesondere 4,4'-Diamino-diphenylmethan oder ein Isomeres hiervon, hydriert.

6. Verfahren nach einem der Ansprüche 1 bis 5,
**dadurch gekennzeichnet,**
**dass** man die Hydrierung bei einer Temperatur im Bereich von 20 bis 200 °C, insbesondere 50 bis 150°C, und einem Wasserstoffpartialdruck im Bereich von 3 bis 30 Mpa, insbesondere 3 bis 10 Mpa, durchführt.

7. Verfahren nach einem der Ansprüche 1 bis 6,
**dadurch gekennzeichnet,**
**dass** man die Hydrierung in einem Festbettreaktor, insbesondere in einem Rohrbündelreaktor, in Rieselbettfahrweise durchführt.

8. Verfahren nach einem der Ansprüche 1 bis 7,
**dadurch gekennzeichnet,**
**dass** man einen geträgerten Katalysator verwendet, dessen Aktivmetall Ruthenium auf einen Träger aufgebracht wurde durch Besprühen des Trägers mit einer verdünnten Rutheniumsalzlösung, insbesondere einer Rutheniumnitrosylnitratlösung, bei einer Temperatur von mindestens 80 °C und anschließende Trocknung.

9. Geträgerter Katalysator, der als Aktivmetall Ruthenium alleine oder zusammen mit mindestens einem Metall des I., VII. oder VIII. Nebengruppe des Periodensystems in einer Menge von 0,01 bis 20 Gew.% Aktivmetalle, bezogen auf den geträgerten Katalysator, auf einen Träger aufgebracht enthält, zur Hydrierung eines aromatischen Amins, das mindestens eine an einen aromatischen Kern gebundene Aminogruppe aufweist,
**dadurch gekennzeichnet,**
**dass** der Katalysatorträger eine BET-Oberfläche im Bereich von größer 30 m²/g bis kleiner 70m²/g aufweist und mehr als 50% des Porenvolumens des Katalysatorträgers Makroporen mit einem Porendurchmesser von größer 50 nm und weniger als 5C% Mesoporen mit einem Porendurchmesser von 2 bis 50 nm sind und dass das auf den Katalysator aufgebrachte Aktivmetall eine Eindringtiefe in den Träger im Bereich von 20 bis 500 µm, insbesondere 25 bis 250 µm, aufweist.

## Claims

1. Process for hydrogenating an aromatic amine having at least one amino group attached to an aromatic nucleus, comprising reacting the aromatic amine with hydrogen in the presence of a supported catalyst containing as active metal ruthenium alone or together with at least one metal selected from transition group I, VII or VIII of the periodic table in an amount of 0.01 to 20 wt.% of active metals, based on the supported catalyst, applied to a support, **characterized in that** the catalyst support has a BET surface area ranging from greater than 30 m²/g to less than 70 m²/g and more than 50% of the pore volume of the catalyst support are macropores having a pore diameter of greater than 50 nm and less than 50% of the pore volume of the catalyst support are mesopores having a pore diameter of 2 to 50 nm, and **in that** the active metal applied to the catalyst has a penetration depth into the support ranging from 20 to 500 µm, in particular 25 to 250 µm.

2. Process according to Claim 1, **characterized in that** the ratio of the surface areas of the active metal, determined by CO pulse chemisorption, and of the catalyst support, determined by BET, is greater than 0.01, in particular 0.03 to 0.3.

3. Process according to Claim 1 or 2, **characterized in that** the support material is selected from crystalline and amorphous oxides and silicates, in particular from Al₂O₃, SiO₂, TiO₂, ZrO₂, MgO, ZnO and aluminosilicates.

4. Process according to any one of Claims 1 to 3, **characterized in that** the catalyst support has a BET surface area ranging from 32 to 67 m²/g, the penetration depth of the active metals is in the range from 50 to 200 µm, the ruthenium quantity is in the range from 0.2 to 3 wt.%, based on the catalyst, and at least 55% of the pore volume of the catalyst support is formed from macropores and less than 45% from mesopores.

5. Process according to any one of Claims 1 to 4, **characterized in that** a 4,4'-diaminodiphenyl-(C₁ to C₄)alkane and/or a 2,4'- or 2,2'-isomer thereof, in particular 4,4'-diaminodiphenylmethane or an isomer thereof is hydrogenated.

6. Process according to any one of Claims 1 to 5, **characterized in that** the hydrogenation is performed at a temperature ranging from 20 to 200°C, in particular 50 to 150°C, and a hydrogen partial pressure ranging from 3 to 30 Mpa, in particular 3 to 10 Mpa.

7. Process according to any one of Claims 1 to 6, **characterized in that** the hydrogenation is performed in a fixed bed reactor, in particular in a tube bundle reactor, in the trickle-bed mode.

8. Process according to any one of Claims 1 to 7, **characterized in that** the supported catalyst used has its active metal ruthenium applied to a support by spraying the support with a dilute ruthenium salt solution, in particular a ruthenium nitrosyl nitrate solution, at a temperature of at least 80 °C and subsequent drying.

9. Supported catalyst which contains as active metal ruthenium alone or together with at least one metal selected from transition group I, VII or VIII of the periodic table in an amount of 0.01 to 20 wt.% of active metals, based on the supported catalyst, applied to a support, for hydrogenation of an aromatic amine having at least one amino group attached to an aromatic nucleus, **characterized in that** the catalyst support has a BET surface area ranging from greater than 30 m²/g to less than 70 m²/g and more than 50% of the pore volume of the catalyst support are macropores having a pore diameter of greater than 50 nm and less than 50% of the pore volume of the catalyst support are mesopores having a pore diameter of 2 to 50 nm, and **in that** the active metal applied to the catalyst has a penetration depth into the support ranging from 20 to 500 µm, in particular 25 to 250 µm.

## Revendications

1. Procédé d'hydrogénation d'une amine aromatique, qui présente au moins un groupe amino lié à un noyau aromatique, comprenant la transformation de l'amine aromatique avec de l'hydrogène en présence d'un catalyseur supporté qui contient comme métal actif du ruthénium, seul ou ensemble avec au moins un métal du Ier, VIIème ou VIIIème groupe secondaire du système périodique en une quantité de 0,01 à 20% en poids de métal actif, par rapport au catalyseur supporté, appliqué sur un support, **caractérisé en ce que** le support de catalyseur présente une surface BET dans une plage supérieure à 30 m²/g à moins de 70 m²/g et plus de 50% du volume des pores du support de catalyseur sont des macropores présentant un diamètre de pores supérieur à 50 nm et moins de 50% sont des mésopores présentant un diamètre de pores de 2 à 50 nm et **en ce que** le métal actif appliqué sur le catalyseur présente une profondeur de pénétration dans le support dans la plage de 20 à 500 µm, en particulier de 25 à 250 µm.

2. Procédé selon la revendication 1, **caractérisé en ce que** le rapport de la surface du métal actif, déterminée par chimisorption pulsée de CO, et de la surface support de catalyseur, déterminée selon BET, est supérieur à 0,01, en particulier de 0,03 à 0,3.

3. Procédé selon la revendication 1 ou 2, **caractérisé en ce que** le matériau support est choisi dans la série des oxydes et silicates cristallins et amorphes, en particulier choisi dans la série Al₂O₃, SiO₂, TiO₂, ZrO₂, MgO, ZnO et les aluminosilicates.

4. Procédé selon l'une quelconque des revendications 1 à 3, **caractérisé en ce que** le support de catalyseur présente une surface BET dans la plage de 32 à 67 m²/g, la profondeur de pénétration des métaux actifs se situe dans la plage de 50 à 200 µm et la quantité de Ru se situe dans la plage de 0,2 à 3% en poids par rapport au catalyseur et au moins 55% du volume de pores du support de catalyseur est formé par des macropores et moins de 45% est formé par des mésopores.

5. Procédé selon l'une quelconque des revendications 1 à 4, **caractérisé en ce qu'**on hydrogène un 4,4'-diaminodiphényl-(C₁ à C₄)alcane et/ou un isomère 2,4' ou 2,2' de celui-ci, en particulier le 4,4'-diaminodiphénylméthane ou un isomère de celui-ci.

6. Procédé selon l'une quelconque des revendications 1 à 5, **caractérisé en ce qu'**on réalise l'hydrogénation à une température dans la plage de 20 à 200°C, en particulier de 50 à 150°C, et à une pression partielle d'hydrogène dans la plage de 3 à 30 MPa, en particulier de 3 à 10 MPa.

7. Procédé selon l'une quelconque des revendications 1 à 6, **caractérisé en ce qu'**on réalise l'hydrogénation dans un réacteur à lit fixe, en particulier un réacteur à faisceau tubulaire, dans un mode opératoire à lit de ruissellement.

8. Procédé selon l'une quelconque des revendications 1 à 7, **caractérisé en ce qu'**on utilise un catalyseur supporté dont le métal actif ruthénium a été appliqué sur un support par aspersion du support avec une solution diluée de sel de ruthénium, en particulier une solution de nitrate de ruthénium-nitrosyle, à une température d'au moins 80°C et séchage consécutif.

9. Catalyseur supporté qui contient comme métal actif du ruthénium, seul ou ensemble avec au moins un métal du Ier, VIIème ou VIIIème groupe secondaire du système périodique en une quantité de 0,01 à 20% en poids de métal actif, par rapport au catalyseur supporté, appliqué sur un support, pour l'hydrogénation d'une amine aromatique qui présente au moins un groupe amino lié sur un noyau aromatique, **caractérisé en ce que** le support de catalyseur présente une surface BET dans une plage supérieure à 30 m²/g à moins de 70 m²/g et plus de 50% du volume des pores du support de catalyseur sont des macropores présentant un diamètre de pores supérieur à 50 nm et moins de 50% sont des mésopores présentant un diamètre de pores de 2 à 50 nm et **en ce que** le métal actif appliqué sur le catalyseur présente une profondeur de pénétration dans le support dans la plage de 20 à 500 µm, en particulier de 25 à 250 µm.
